# EUROPEAN PATENT APPLICATION

(11) **EP 1 443 118 A1**
(43) Date of publication of application: **04.08.2004**
(21) Application number: 03028521.7
(22) Date of filing: 10.12.2003
(51) Int. Cl.: C12Q 1/68

(54) **Primers for detecting Fusobacterium nucleatum by PCR methods and methods for detection**

(30) Priority: 10.12.2002 JP 2002358698; 02.12.2003 JP 2003403715
(71) Applicant: Kao Corporation, Tokyo 103-8210 (JP)
(72) Inventor: Iwase, Tadayuki, Kao Corporation Res. Laboratories, Tokyo 131-8501 (JP); Itano, Morihide, Kao Corporation Res. Laboratories, Tokyo 131-8501 (JP); Yano, Yoshitaka, Kao Corporation Res. Laboratories, Tokyo 131-8501 (JP)
(74) Representative: VOSSIUS & PARTNER

(57) **Abstract**

The present invention provides a method for specifically detecting or identifying Fusobacterium nucleatum in a biological sample, which is a suppurative disease-related bacterium and also is a halitosis-causing bacterium. The method employs the following primers (1) and (2): namely, (1) a forward primer with a consecution of 10 or more nucleotides in length, having a nucleotide sequence contained in a sequence of SEQ ID No. 1; and, (2) a reverse primer with a consecution of 10 or more nucleotides in length, having a nucleotide sequence contained in a sequence complementary to SEQ ID No. 2.

## Description

### Field of the Invention

The present invention relates to a method for specifically detecting or quantifying Fusobacterium nucleatum, which is a suppurative disease-related bacteria and also is a halitosis-causing bacteria.

### Background of the Invention

In general, detection of microorganisms such as bacterium has been conducted by cultivation such as proliferative cultivation or separate cultivation, and it is required to make single colonies grow, whereby taking several days or several weeks. Moreover, the isolated microorganisms is further proliferated in order to conduct morphological observation, cell staining such as Gram staining, investigation of various biochemical characteristics including propionic acid production test, saccharide catabolism test and the like. Thus, the identification of microorganisms employing the cultivation method has involved facility-dependency, time-consuming and less cost-efficient.

As a method to overcome defects of the cultivation method, a PCR (polymerase chain reaction) method has recently been employed for the identification of microorganisms. The PCR method permits a rapid and high sensitive identification of microorganisms. However, the PCR method needs to use primers, and detection or identification of a target microorganism is dependent on designs of the primers.

On the other hand, as bacteria of the Fusobacterium genus which can be isolated from a human oral cavity, there has been known Fusobacterium naviforme, Fusobacterium periodonticum, Fusobacterium necrophorum, Fusobacterium varium, Fusobacterium russii, Fusobacterium nucleatum (hereinafter referred to as Fn microorganism) and the like.

The Fn microorganism, which is also known as a halitosis-causing bacterium, decomposes a sulfur-containing amino acid and a peptide containing such a sulfur-containing amino acid which are present in an oral food debris or detached mucosa to produce volatile sulfide compounds (hereinafter referred to as VSC). To the contrary, the VSC production ability of any bacteria of the Fusobacterium genus other than the Fn microorganism is not as high as that of the Fn microorganism. In addition, the Fn microorganism is suggested to be related with periodontal diseases since it has a specific ability of being coaggregated with periodontal pathogens such as Porphyromonas gingivalis and Prevotella intermedia.

Accordingly, the Fn microorganism is a particularly important strain among the bacteria of the Fusobacterium genus. Nevertheless, the Fn microorganism is very difficult to be isolated for the culture, since it has a remarkably analogous relationship with other bacteria of the Fusobacterium genus, and no selective medium capable of specifically detecting the Fn microorganism has been developed.

As an attempt to detect and identify an Fn microorganism, a method for detecting a bacterium was reported in which the 16S ribosomal DNA (hereinafter referred to as 16S rDNA) of a test microorganism was extracted and a PCR is conducted using, as primers, the oligonucleotides consisting of several ten nucleotide sequences complementary to parts of this DNA (see Conrads, G. et al., J. Endodontics. 25:433-438, 1997). Nevertheless, the conventional PCR method has not been able to detect the Fn microorganism specifically.

### Summary of the Invention

The present invention provides a method for detecting and/or quantifying Fusobacterium nucleatum by a PCR method employing the following primers (1) and (2):
(1) a forward primer, comprising a nucleotide sequence which is depicted as a portion of SEQ ID No. 1 and has at least ten nucleotides in length;
(2) a reverse primer, comprising a nucleotide sequence which is depicted as a portion of a sequence complementary to SEQ ID No.2 and has at least ten nucleotides in length.

### Detailed Description of the Invention

The present invention provides a simple and rapid detection method which is capable of distinguishing an Fn microorganism specifically among the bacteria of the Fusobacterium genus and which also capable of distinguishing from human-derived DNAs contaminating a biological sample.

Inventors of the present invention investigated the ribosomal DNA of the Fn microorganism to find that the 16S rDNAs are remarkably analogous between closely related species among the Fusobacterium genus, and therefore it is difficult to detect or identify the Fn microorganism by a PCR method simply using primers prepared so as to have a nucleotide sequence in a region of 16S rDNA. On the other hand, a 23S ribosomal DNA (hereinafter referred to as 23S rDNA) is analogous with the human gene DNA, and therefore it is not desirable for a case of analyzing a biological sample such as a supragingival plaque, subgingival plaque, tongue plaque, saliva, gingival crevice fluide, blood, cerebrospinal fluid, suppurative focus or other tissues.

Hence, in the present invention, a PCR method is conducted in a combination use of "a forward primer with a consecution of 10 or more nucleotides" which has a nucleotide sequence contained in the 16S rDNA and/or an adjacent spacer (a region represented by SEQ ID No. 1) and "a reverse primer with a consecution of 10 or more nucleotides" which has a nucleotide sequence contained in the 23S rDNA (a sequence complementary to a region depicted as SEQ ID No. 2). Thus, such a combination use makes it possible to achieve a simple and rapid detection, which is capable of specifically distinguishing the Fn microorganism and also capable of distinguishing from human-derived DNAs contaminating a biological sample.

Brief descriptions of the accompanying drawings are as follows:
FIG. 1 shows the structure of the DNA encoding an rRNA;
FIG. 2 shows the results of the electrophoresis of a PCR amplification product of Fusobacterium nucleatum in Example 1;
FIG.3 shows the results of the electrophoresis of amplification products of the bacteria of the Fusobacterium genus other than Fusobacterium nucleatum and also of human-derived cells;
FIG. 4 shows the results of PCRs of biological samples including (1) saliva, (2) tongue plaque, (3) subgingival plaque and (4) oral mucosa; and,
FIG. 5 shows the results of a semi-nested PCR of a biological sample.

In the present invention, a forward primer comprising a nucleotide sequence which is depicted as a portion of SEQ ID No.1 and has at least 10 nucleotides in length has, as its partial or entire sequence, a nucleotide sequence which relates to SEQ ID No.1 and has at least 10 nucleotides in length.

More specifically, the forward primer has, at its 3' terminal, a nucleotide sequence having at least 10 nucleotides and being same as a part of SEQ ID No. 1, and its 5' terminal may be extended by an additional sequence not relating to any part of SEQ ID No. 1.

It is not limited to specific ones with proviso that it is capable of hybridizing with a complementary strand of SEQ IDNo. 1 in a condition of the PCR method described hereaf ter, and not capable of hybridizing with a DNA derived from a normal human gingival fibroblast cell.

The forward primer is preferably designed so as to adapt to the following restriction: namely, it is preferable for a length of the primer to be in a range of 10 nucleotides or more and 40 nucleotides or less, more preferably 12 nucleotides or more and 30 nucleotides or less, and even more preferably 15 nucleotides or more and 25 nucleotides or less. The primer may be designed so as to have a value of Tm within a preferable range of 40 °C or more and 65 °C or less, and more preferably 50 °C or more and 60 °C or less. GC content is preferably in a range of 25 % or more and 70 % or less, and more preferably 40 % or more and 60 % or less.

Preferable examples of the forward primer include:

GTTTGATCCTGGCTCAG (SEQ ID No. 11); CTTAACACATGCAAGTC (SEQ
ID No. 12); AATGCTTAACACATGCAAGTC (SEQ ID No. 13);
TCCTACGGGAGGCAGCAGT (SEQ ID No. 14); and, GTCTTGTACACACCGCCC
(SEQ ID No. 15).

A reverse primer, comprising a nucleotide sequence which is depicted as a portion of a sequence complementary to SEQ ID No.2 and has at least ten nucleotides in length has, as its partial or entire sequence, a nucleotide sequence which relates to SEQ ID No.2 and has at least 10 nucleotides in length.

More specifically, the reverse primer has, at its 3' terminal, a nucleotide sequence having at least 10 nucleotides and being complementary to a part of SEQ ID No. 2, and its 5' terminal may be extended by an additional sequence not relating to any part of SEQ ID No. 2.

It is not limited to specific ones with proviso that it is capable of hybridizing with a DNA strand represented by SEQ ID No. 2 in a condition of the PCR method described hereafter, and not capable of hybridizing with the 23S rDNA derived from any of Fusobacterium periodonticum (ATCC33693), Fusobacterium necrophorum (ATCC25286), Fusobacterium varium (ATCC8501).

The reverse primer is preferably designed so as to adapt to the following restriction: namely, it is preferable for a length of the primer to be in a range of 10 nucleotides or more and 40 nucleotides or less, more preferably 12 nucleotides or more and 30 nucleotides or less, and even more preferably 15 nucleotides or more and 25 nucleotides or less.

The primer may be designed so as to have a value of Tm within a preferable range of 40 °C or more and 65 °C or less, and more preferably 50 °C or more and 60 °C or less. GC content is preferably in a range of 25 % or more and 70 % or less, and more preferably 40 % or more and 60 % or less.

Preferable examples of the reverse primer include:

GCCATCACCCAAATGG (SEQ ID No. 16); and, AAGAAGGGTAACCGACTT
(SEQ ID No. 17).

In particular, it is preferable to employ a primer set using a combination of either the following primers (A) and (B) or the following primers (B) and (C) in a viewpoint of specifically detecting or identifying the Fn microorganism from a biological sample.
(A) a primer, comprising a nucleotide sequence which is depicted as a portion or a whole of a region between nucleotides 1 to 39 of SEQ ID No.3 and has at least ten nucleotides in length;
(B) a primer, comprising a nucleotide sequence which is depicted as a portion or a whole of a sequence complementary to a region between nucleotides 863 to 883 of SEQ ID No.3 and has at least ten nucleotides in length;
(C) a primer, consisting of a nucleotide sequence which is depicted as a region between nucleotides 1 to 18 of SEQ ID No.3.

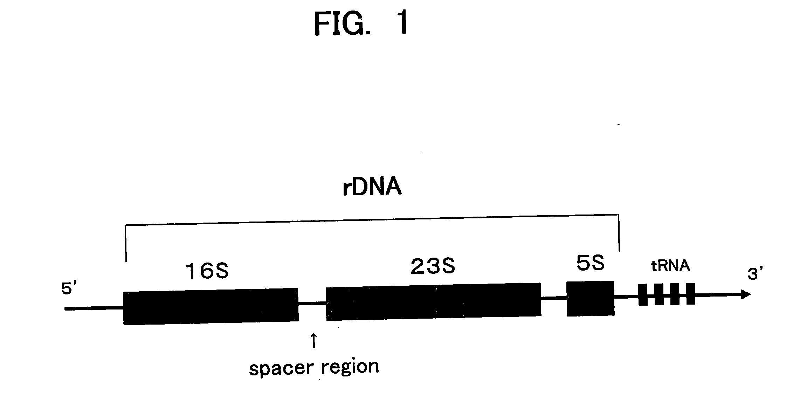

Since a PCR method is conducted using a biological sample containing not only a Fn microorganism-derived DNA but also a human-derived DNA, a nucleotide sequence of nucleotides 1 to 39 of SEQ ID No. 3, which is one of nucleotide sequences of the primer (A) , is found as a preferable region for distinguishing the DNAs between ordinary bacteria and human. This nucleotide sequence was revealed to be present at a site consisting of 5 nucleotides at the 3' terminal of the 16S rDNA and the 5' terminal side of a spacer region which is formed between the 16S rDNA and the 23S rDNA(See FIG. 1).

Subsequently, the above-mentioned oligonucleotide primers for the amplification by a PCR method were designed and synthesized. When these primers are used in the PCR of a biological sample containing the Fn microorganism, the amplification products specific for the Fn microorganism are exclusively obtained.

As a sample usable for detection or identification of the Fn microorganism, there may be exemplified oral cavity-related biological samples such as a supragingival plaque, subgingival plaque, tongue plaque, saliva, gingival crevice fluide and the like. In addition, there may also be used other biological samples such as blood, cerebrospinal fluid, suppurative focus or other tissues. The oligonucleotides to be used as primers may be synthesized chemically or may be derived from a natural products.

The primers (A), (B) and (C), which are preferably used in a PCR method according to the present invention, are discussed below more in detail. In conducting a PCR, an oligonucleotide which has a nucleotide sequence, as its partial or entire sequence, being depicted as a portion or a whole of a region between nucleotides 1 to 39 of SEQ ID No.3 and having at least ten nucleotides in length and which is defined as a nucleotide sequence of the primer (A), is used as a forward primer, while an oligonucleotide which has a nucleotide sequence, as its partial or entire sequence, being depicted as a portion or a whole of a sequence complementary to a region between nucleotides 863 to 883 of SEQ ID No.3 and having at least ten nucleotides in length and which is defined as a nucleotide sequence of the primer (B), is used as a reverse primer. The chain length of the each primer is preferably 10 nucleotides or more and 40 nucleotides or less, more preferably 12 nucleotides or more and 30 nucleotides or less, and even more preferably 15 nucleotides or more and 25 nucleotides or less.

As the primer (A), there may be used for example: the primer (C) having a nucleotide sequence consisting of the following 18 nucleotides AACGTGCGGATGGATCAC; a nucleotide sequence of nucleotides 1 to 22 of SEQ ID No.3, namely AACGTGCGGATGGATCACCTCC; a nucleotide sequence of nucleotides 7 to 26 of SEQ ID No.3, namely CGGATGGATCACCTCCTTTC; a nucleotide sequence of nucleotides 12 to 31 of SEQ ID No.3, namely GGATCACCTCCTTTCTAAGG; and, a nucleotide sequence of nucleotide 16 to 33 of SEQ ID No.3, namely CACCTCCTTTCTAAGGAG.

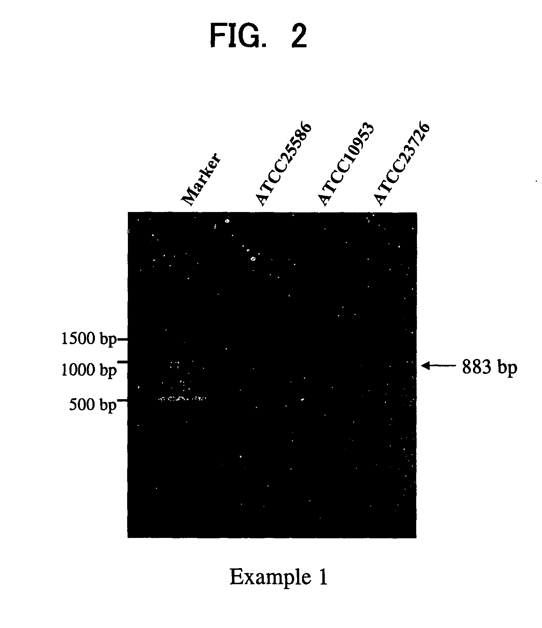

In a case of conducting a PCR using one kind of the primer (B) such as a primer consisting of a nucleotide sequence complementary to a region between nucleotides 863 to 883 of SEQ ID No. 3 and the primer (C), the amplification product of the nucleotide sequence represented by SEQ ID No. 3 can specifically be obtained. The length of a major amplification product is calculated to be 883 bp, and a band of about 900 bp indicating the amplification product can be observed in an electrophoresis image (see FIG. 2).

When the cell count of the Fn microorganism in a sample is extremely small, or when a further clarified electrophoresis image is intended, then a semi-nested PCR method is preferably conducted with the use of the three primers (A), (B) and (D). The primer (D) has a nucleotide sequence, as its partial or entire sequence, being depicted as a portion or a whole of a sequence complementary to a region between nucleotides 124 to 156 of SEQ ID No.3 and having at least ten nucleotides in length. A nucleotide sequence of the primer (D) is present in a site called as a spacer region between the 16S rDNA and the 23S rDNA. The nucleotide sequence of the primer (D) may be consist of 29 nucleotides, and preferably in a range from 15 nucleotides to 25 nucleotides, and there may be used a primer (E) having a nucleotide sequence consisting of 20 nucleotides, as the primer (D). The primer (E) consists of a nucleotide sequence depicted as a sequence complementary to a region between nucleotides 124 to 143 of SEQ ID No.3.

The semi-nested PCR method comprises two stages of amplification. In the first stage of amplification, an amplification product containing a target region is obtained. Then, the resultant amplification product is used as a template to conduct the second stage of amplification. In this second stage, either one of the forward and the reverse primers (namely, inner primers) to be used is capable of positioning at a site inner than a site where either one of the primers (namely, outer primers) used in the first stage positions, whereby excluding the amplification products derived from the DNA in a region other than the target region.

In a case of using the primers (A), (B) and (D), the first stage of amplification in the PCR is conducted using the primers (A) and (B) as outer primers. Then, using a part of the sample thus amplified in the first stage, the second stage of amplification is initiated. In the second stage of amplification, the PCR is conducted using the primers (A) and (D) as inner primers. Since a major amplification product in such a case is dependent on the primers employed in the second amplification stage, it corresponds to a part of the nucleotide sequence represented by SEQ ID No. 3.

As to the outer primers usable in the first amplification stage, oligonucleotides other than the primer (A) or (B) may be used. For example, an oligonucleotide designed based on the nucleotide sequence of the 16S rDNA such as CGTCACACCACGAGAGTTGG (namely, nucleotides 1384 to 1403 of SEQ ID No. 1) and the like can be used instead of the primer (A). Also instead of the primer (B), an oligonucleotide having a nucleotide sequence complementary to a nucleotide sequence designed based on the 23S rDNA such as CCATTTGGGTGATGGC (namely, nucleotide 597 to 612 of SEQ ID No.2 ) can be used as the outer primer.

The primers according to the present invention can be applied also to a nested PCR method. The nested PCR method is different from the semi-nested PCR method in that it uses an inner primer set in which respective inner primers to be used for the both sides are capable of positioning at inner sites on the target region rather than the outer primers.

In addition to the primers (C) and (B), the followings are exemplified as the outer primers which can be used in the nested PCR.

Forward primer: an oligonucleotide which has a nucleotide sequence, as its partial or entire sequence, being depicted as a portion of a region between nucleotides 1 to 123 of SEQ ID No.3 wherein the portion related to the region of SEQ ID No.3 has at least ten nucleotides in length, or being depicted as a portion of SEQ ID No. 4 wherein the portion related to SEQ ID No. 4 has at least ten nucleotides in length.

Reverse primer: an oligonucleotide which has a nucleotide sequence, as its partial or entire sequence, being depicted as a portion of the 23S rDNA(SEQ ID No.2).

For example, the first stage of amplification can be conducted using, as outer primers, a primer with a sequence of CGTCACACCACGAGAGTTGG (nucleotides 1384 to 1403 of SEQ ID No. 1) designed based on the nucleotide sequence of the 16S rDNA and, a primer with a complementary nucleotide sequence corresponding to AAGAAGGGTAACCGACTT (nucleotides 1256 to 1273 of SEQ ID No. 2) among the nucleotide sequence of the 23S rDNA. In such a case, the primer set of (A) and (B) or the primer set of (B) and (D), all of which are inventive primers, is preferably used as the inner primers for the purpose of specifically detecting or identifying an Fn microorganism.

The PCR method is a method to amplifying genes commonly practicedby one skilled in the art, and fundamental techniques are described below briefly.

A double-stranded DNA used as a template is separated into single-stranded DNAs by heating (the thermal denaturation). Then the annealing process is carried out so as to sandwich a target region of the each single-stranded DNA by hybridized sites in such manner that oligonucleotides referred to as primers are hybridized with sites of the 5' terminal of the respective single-stranded DNAs which have separated by the aforementioned thermal denaturation process and have a complementary relationship with respect to the separated counter part. When a Taq DNA polymerase is made to act in the presence of 4 kinds of dNTP (deoxyribonucleotide triphosphates) as substrates, the nucleotides are added to the 3' terminal of the primer in accordance with the nucleotide sequence of the template, resulting in the extension of the DNA chain (extension reaction). By repeating this reaction, a large amount of the DNA fragment containing the target region can be obtained. For the reaction of primer-synthesis or chain-extension, a dUTP may be used as the substrate instead of the dTTP among the dNTPs.

In the present invention, the PCR method preferably employs the following temperature condition in a single cycle: a thermal denaturation for converting a double-stranded DNA into a single strand is carried out at 90 to 98 °C; an annealing reaction for hybridizing the primer with the template DNA is carried out at 37 to 65 °C; and, an extension reaction for allowing a Taq DNA polymerase to exert the effect is carried out at 50 to 75 °C. By repeating this cycle for several ten times, a target sequence can be amplified. After the PCR, the amplification product is separated for example by an electrophoresis, subjected to a nucleic acid staining for example with ethidium bromide, and then can give a judgement that the microorganism to be detected is present in the sample if the chain length of the amplified polynucleotide sequence is equal to the chain length of the target sequence. For detecting the amplified polynucleotide sequence, a high performance liquid chromatography is also effective.

When an additional sequence extends from the 5' terminal of an essential part of the nucoleotide sequence ( For example, essential for the primer (A) is a sequence which is depicted as a portion or a whole of a region between nucleotides 1 to 39 of SEQ ID No. 3 and has at least ten nucleotides in length, or essential for the primer (B) is a sequence which is depicted as a portion or a whole of a sequence complementary to a region between nucleotides 863 to 883 of SEQ ID No.3 and has at least ten nucleotides in length), the additional portion in the primer chain length is imparted to an amplification product. To the contrary, the use of one having a shorter 5' terminal results in an amplification product which is shorter by such a deleted portion. The primers may be labeled with one or more selected from biotin, digoxigenin, FITC (fluorescein isothiocyanate), acridine, dinitrophenyl, alkaline phosphatase, luciferase and [³²P]dNTP.

While various biological sample may be suggested as a sample to be subjected to the invention, those which may be exemplified are saliva, tongue plaque, supragingival plaque, subgingival plaque, oral mucosa, gingival crevice fluid, blood, cerebrospinal fluid, suppurative focus sample, feces, urine or cultured materials of these biological materials.

A biological sample thus obtained can be subjected to a PCR directly or after a DNA extraction. The DNA extraction may be conducted by a conventional method. For example, the DNA extraction may employ one or more of an alkaline extraction or surfactant extraction, enzyme treatment, boiling and the like, and may also employ a commercial kit.

A sample containing the DNA extracted froman organism's body may further be purified by a conventional method. For example, the DNA purification may employ one or more of a high performance liquid chromatography or alcohol precipitation, salting-out, silica gel column, silica gel membrane and the like, and may also employ a commercial kit.

The primers and the amplification products are also useful as probes for detecting an Fn microorganism. The amplification products may be converted into DNA fragments having suitable lengths using restriction enzymes and the like. Such restriction enzymes are one or more of RcoR I, Mse I, Ban II, Alu I, Mbo I, Fok I, Taq I, Mbo II, Hinf I and Ava II. A DNA fragment obtained by a restriction enzyme treatment is preferably purified by means of an electrophoresis, high performance liquid chromatography, silica gel column, silica gel membrane, nylon membrane and the like.

Any of these probes can be labeled with a label. The probe may be labeled with one or more of biotin, digoxigenin, FITC(fluoresceinisothiocyanate),acridine,dinitrophenyl, alkaline phosphatase, luciferase or [³²P]dNTP.

Any of these probe can be bound to a support and employed as a DNA chip. The method for producing a DNA chip may be any conventional method. For example, it may employ a probe alignment type in which a probe is aligned on a chip substrate, or a probe synthesis type in which a DNA extension reaction is performed directly on a substrate such as a glass or silicon to form a probe DNA. Alternatively, a commercial DNA chip producing device may be employed, and the reading may be effected using a DNA chip reader.

A kit for detecting and identifying a microorganism comprises proves and/or primers according to the present invention. It may also contain other components such as a Taq DNA polymerase, other enzymes, substrates such as dNTPs and the like, as well as buffer solutions.

### EXAMPLES

The present invention is further described in the following Examples which are not intended to restrict the invention.

### [Example 1] Fn microorganism detection 1

Using the following primers, a standard microorganism of Fusobacterium nucleatum was detected.

Forward primer: 5'-AACGTGCGGATGGATCAC-3'

Reverse primer: 5'-CTACGCCAAACGACTAATTCG-3'

Three types of standard microorganisms of Fusobacterium nucleatum (Fusobacterium nucleatum ATCC25586, ATCC10953, ATCC23726) were incubated anaerobically in a modified FMmedium (available from NISSUI PHARMACEUTICAL CO., LTD.) at 37 °C for 3 to 5 days, and the colonies formed were taken at one supatula-full amount of platinum loop and processed using a commercial kit (QIAGEN K.K., DNA mini-kit) to obtain a DNA extract. 1 µl of the DNA extract was combined with 1.5 µl of 50 mM MgCl₂ solution, each 2 µl of 2 mM dNTP solutions (Applied Biosystems) , 1 µl of a 12.5 pmol/µl forward primer solution, 1 µl of a 12.5 pmol/µl reverse primer solution, 0.5 µl of a 5 U/µl Taq DNA polymerase solution (Applied Biosystems) , 5 µl of an Ampli Taq Gold buffer solution (Applied Biosystems) and a sterilized ultrapure water at a volume to obtain the total volume of 50 µl, thereby obtaining a reaction solution.

Conditions of the PCR reaction was as described below, and the cycle of the PCR employing such conditions was repeated 40 times.
Thermal denaturation: 94 °C, 30 seconds.
Annealing: 55 °C, 30 seconds.
Extension reaction: 72 °C, 30 seconds.

The procedure was conducted using a GeneAmp 9700 system of Applied Biosystems. The presence or absence of the amplification products was determined by a conventional agarose electrophoresis. The agarose gel for the electrophoresis was provided, and placed in a running device which had previously been filled with a TAE (tris-acetate, ethylenediamine tetraacetic acid) buffer solution, and then the PCR reaction product was applied onto the sample groove. After running for 15 minutes at 100 V, the agarose gel was soaked in an ethidium bromide solution for 30 minutes to stain the nucleic acid. After the staining, the bands of the amplification products were identified using an UV transilluminater. The results of the electrophoresis are shown in FIG. 2. In the figure, Lane 1 represents a marker, Lane 2 represents ATCC25586 strain, Lane 3 represents ATCC10953 strain and Lane 4 represents ATCC23726 strain. All of the three microorganisms of Fusobacterium nucleatum exhibited the band representing the amplification product of about 900 bp.

### [Example 2] Fn microorganism detection 2

Using the primers shown below, the test was performed similarly to Example 1.

The primers employed in this Example are shown below.

Forward primer: 5'-GGATTAGATACCCTGGTAGTC-3'

Reverse primer: 5'-GCCATCACCCAAATGG-3'

Fusobacterium nucleatum (ATCC25586 strain)exhibited the band representing the amplification product of about 1500 bp.

### [Comparative 1] PCR of Fusobacterium genus bacteria other than Fn microorganism and human-derived cell

Test bacteria and test cells subjected are 5 bacteria other than the Fn microorganism, namely, F.naviforme (ATCC25832), F.periodonticum (ATCC33693), F.necrophorum (ATCC25286), F.varium (ATCC8501), F.russii (ACTT25533) as well as normal human gingival fibroblast (ATCC CRL1292).

The normal human gingival fibroblast was incubated for 72 hours at 37 °C under an atmosphere of 5% CO₂ in a modified EAGLE medium (available from Dulbecco) supplemented with 10% fetal bovine serum, and then recovered, washed with a physiological saline, and then nucleic acid was isolated. Otherwise, the bacteria strains were processed similarly to Example 1.

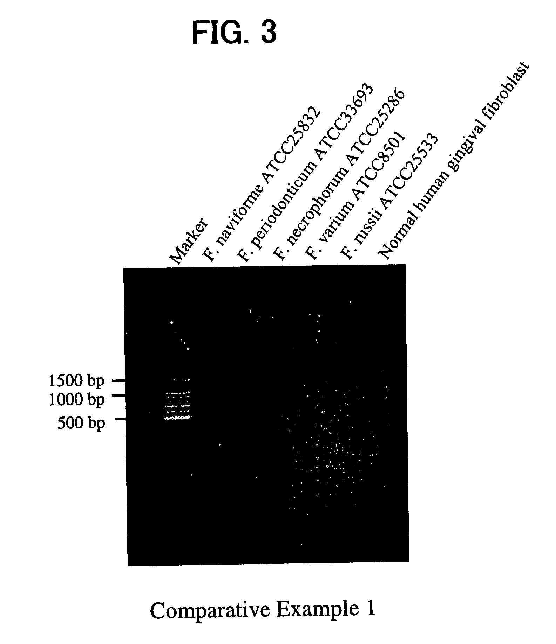

The results of the electrophoresis are shown in FIG. 3. In the electrophoresis images, Lane 1 represents a marker, Lane 2 represents F.naviforme, Lane 3 represents F.periodonticum, Lane 4 represents F.necrophorum, Lane 5 represents F.varium, Lane 6 represents F.russii, and Lane 7 represents the normal human gingival fibroblast. No lane exhibited a band showing the amplification product.

### [Example 3] PCR of biological samples

### 1. Preparation of samples

Saliva, tongue plaque, subgingival plaque and oral mucosa were taken as biological samples from 5 subjects. The sampling method is as described below.

### (1) Saliva

1 ml of saliva was taken and centrifuged at 5000 g for 5 minutes. The supernatant was discarded, and the residue was combined with 1 ml of PBS (phosphate buffered saline) and resuspended. This procedure was repeated three times and the sample after being washed was subjected to the DNA extraction.

### (2) Tongue plaque

A lingual brush was used to rub the tongue to collect the tongue plaque. A 10 mg aliquot of the tongue plaque was combined with 1 ml of PBS, suspended and centrifuged at 5000 g for 5 minutes. The supernatant was discarded, and the residue was combined with 1 ml of new PBS, resuspended, and centrifuged again at 5000 g for 5 minutes. This procedure was repeated three times, and then the sample after being washed was subjected to the DNA extraction.

### (3) Subgingival plaque

2 Paper points (available from United Dental Manufacturers Inc) were inserted into a single periodontal pocket to collect the subgingival plaque. These paper points were shaken under vortex vigorously for 1 minute in 1 ml of PBS to recover the deposited materials. The paper points were removed and the suspension was centrifuged at 5000 g for 5 minutes. The supernatant was discarded, and the residue was combined with 1 ml of PBS to resuspend the sample, which was centrifuged at 5000 g for 5 minutes. This procedure was repeated three times, and the sample after being washed was subjected to the DNA extraction.

### (4) Oral mucosa

A seed swab was used to collect a buccal mucosa. The collected material was suspended in 1 ml of PBS. The suspension was centrifuged at 5000 g for 5 minutes. The supernatant was discarded, and the residue was combined with 1 ml of PBS to resuspend the sample, which was centrifuged at 5000 g for 5 minutes. This procedure was repeated three times and the sample after being washed was subjected to the DNA extraction.

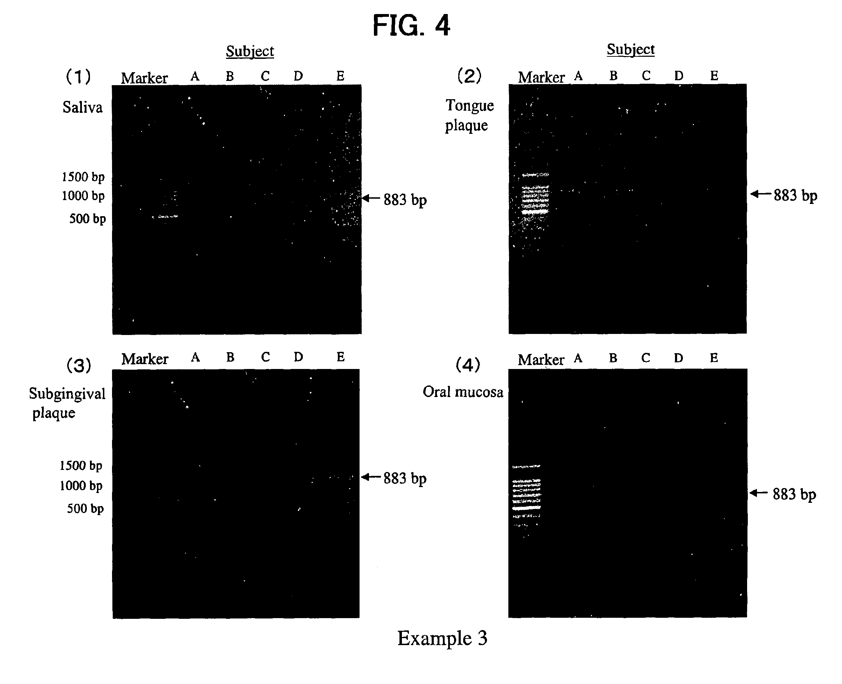

Otherwise, the procedure similar to that in Example 1 was employed. The results of the electrophoresis are shown in Figures 4 (1) to 4 (4). FIG. 4 (1) shows the results of the detection from the saliva, FIG. 4(2) shows the results of the detection from the tongue plaque, FIG. 4(3) shows the results of the detection from the subgingival plaque and FIG. 4(4) shows the results of the detection from the oral mucosa. In each figure which shows the electrophoresis images of respective collected samples, Lane 1 represents a marker, Lane 2 represents Subject A, Lane 3 represents Subject B, Lane 4 represents Subject C, Lane 5 represents Subject D and Lane 6 represents Subject E. About 900 bp band was observed in 3 out of 5 subjects with the saliva, 4 out of 5 with the tongue plaque, all of 5 with the subgingival plaque and 1 out of 5 with the oral mucosa.

### [Example 4] Quantitative PCR of biological samples

Using the subgingival plaque samples subjected in Example 3, a quantitative PCR was conducted. As a nucleic acid assay reagent, 5 µl of an SYBR Green (available from Molecular Probe) which had been subjected to a 3000-fold dilution was used. 1 µl of the primers described below which had been diluted to 12.5 pM with a sterilized ultrapure water was used.

Forward primer: 5'-AACGTGCGGATGGATCAC-3'

Reverse primer: 5'-TCTAAAGAAATTGTTTAGAG-3'

Other reagents were similar to the reaction solution components in Example 1. They were mixed to prepare a reaction solution, which was then subjected to a quantitative PCR using an ABI prism 7000 system of Applied Biosystems. The results are shown in Table 1.

**Table 1**

| Subject | Bacterial count (copies/collection site) |
|---|---|
| A | 1.9E + 04 |
| B | 5.8E + 06 |
| C | 2.5E + 04 |
| D | 3.1E + 02 |
| E | 8.0E + 01 |

### [Example 5] Semi-nested PCR of biological sample

Using the primers shown below, a semi-nested PCR consisting of a 2-stage PCR was conducted.

Primers for 1'st stage PCR:

Forward primer: 5'-AACGTGCGGATGGATCAC-3'

Reverse primer: 5'-CTACGCCAAACGACTAATTCG-3'

Primers for 2'nd stage PCR:

Forward primer: 5'-AACGTGCGGATGGATCAC-3'

Reverse primer: 5'-TCTAAAGAAATTGTTTAGAG-3'

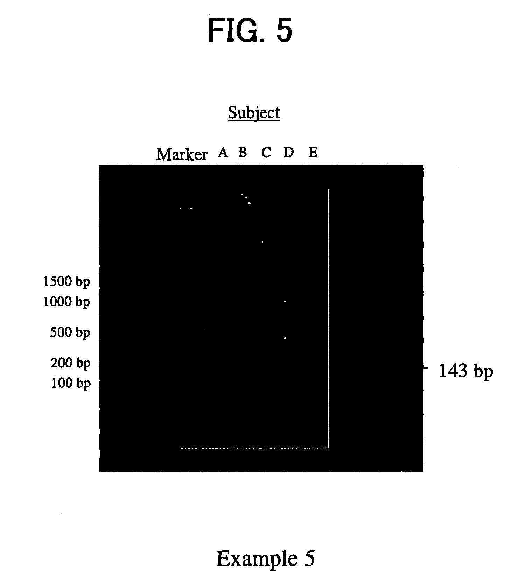

AS a biological sample, the saliva in Example 3 was employed. Both of the PCRs in the 1'st and 2' nd stages involved 40 cycles of the amplification. Otherwise, the procedure similar to that in Example 1 was conducted. The results of the electrophoresis are shown in FIG. 5. In the figure which shows the electrophoresis images of respective collected samples, Lane 1 represents a marker, Lane 2 represents Subject A, Lane 3 represents Subject B, Lane 4 represents Subject C, Lane 5 represents Subject D and Lane 6 represents Subject E. A band showing the amplification product was observed in 3 out of 5 subjects.

### [Example 6] Nested PCR of biological sample

Using the primers shown below, a nested PCR consisting of a 2-stage PCR was conducted. The results were similar to those in Example 5.

Primers for 1'st stage PCR:

Forward primer: 5'-CGTCACACCACGAGAGTTGG-3'

Reverse primer: 5'-CTACGCCAAACGACTAATTCG-3'

Primers for 2'nd stage PCR:

Forward primer: 5'-AACGTGCGGATGGATCAC-3'

Reverse primer: 5'-TCTAAAGAAATTGTTTAGAG-3'

### [Example 7] DNA chip employing Fn microorganism-specific probe

A probe obtained in Example 1 was labeled fluorescently by a standard method, and adsorbed on a glass plate. The plate was reacted with the DNA obtained from the saliva sample in Example 3. The results are shown in Table 2.

**Table 2**

| Subject | Fn microorganism detection |
|---|---|
| A | Detected |
| B | Detected |
| C | Detected |
| D | Not detected |
| E | Not detected |

### [Example 8] Detection kit employing Fn microorganism-specific probe

A probe obtained in Example 1 was labeled fluorescently by a standard method, and adsorbed on glass beads. The beads were reacted with the DNA obtained from the saliva sample in Example 3, and then allowed to develop a color by a standard method. The results are shown in Table 3.

**Table 3**

| Subject | Fn microorganism detection |
|---|---|
| A | Detected |
| B | Detected |
| C | Detected |
| D | Not detected |
| E | Not detected |

## Claims

1. A method for detecting and/or quantifying Fusobacterium nucleatum by a PCR method, **characterized in that** the method employs the following primers (1) and (2):
(1) a forward primer, comprising a nucleotide sequence which is depicted as a portion of SEQ ID No. 1 and has at least ten nucleotides in length;
(2) a reverse primer, comprising a nucleotide sequence which is depicted as a portion of a sequence complementary to SEQ ID No.2 and has at least ten nucleotides in length.

2. A primer set for detecting and/or quantifying Fusobacterium nucleatum by a PCR method, **characterized in that** the primer set comprises the following primers (A) and (B):
(A) a primer, comprising a nucleotide sequence which is depicted as a portion or a whole of a region between nucleotides 1 to 39 of SEQ ID No.3 and has at least ten nucleotides in length;
(B) a primer, comprising a nucleotide sequence which is depicted as a portion or a whole of a sequence complementary to a region between nucleotides 863 to 883 of SEQ ID No.3 and has at least ten nucleotides in length.

3. A primer set for detecting and/or quantifying Fusobacterium nucleatum by a PCR method, **characterized in that** the primer set comprises the following primers (C) and (B) :
(C) a primer, consisting of a nucleotide sequence which is depicted as a region between nucleotides 1 to 18 of SEQ ID No.3;
(B) a primer, comprising a nucleotide sequence which is depicted as a portion or a whole of a sequence complementary to a region between nucleotides 863 to 883 of SEQ ID No.3 and has at least ten nucleotides in length.

4. A gene amplification product specific for Fusobacterium nucleatum obtained by conducting a PCR method using a primer set, **characterized in that** the primer set comprises either the following primers (A) and (B) or the following primers (B) and (C) together with a ribosomal DNA of Fusobacterium nucleatum as a template:
(A) a primer, comprising a nucleotide sequence which is depicted as a portion or a whole of a region between nucleotides 1 to 39 of SEQ ID No.3 and has at least ten nucleotides in length;
(B) a primer, comprising a nucleotide sequence which is depicted as a portion or a whole of a sequence complementary to a region between nucleotides 863 to 883 of SEQ ID No.3 and has at least ten nucleotides in length;
(C) a primer, consisting of a nucleotide sequence which is depicted as a region between nucleotides 1 to 18 of SEQ ID No.3.

5. The gene amplification product according to claim 4 wherein the gene amplification product is a probe labeled with a labeling substance.

6. The gene amplification product according to claim 5, wherein the labeling substance is at least one selected from the group consisting of biotin, digoxigenin, FITC, acridine, dinitrophenyl, luciferase, alkaline phosphatase and [³²P]dNTP.

7. A method for detecting and/or quantifying Fusobacterium nucleatum, **characterized in that** the method comprises:
a first step of conducting a PCR method using primers containing the following primers (A) and (B),
(A) a primer, comprising a nucleotide sequence which is depicted as a portion or a whole of a region between nucleotides 1 to 39 of SEQ ID No.3 and has at least ten nucleotides in length;
(B) a primer, comprising a nucleotide sequence which is depicted as a portion or a whole of a sequence complementary to a region between nucleotides 863 to 883 of SEQ ID No.3 and has at least ten nucleotides in length; and,
a second step of conducting a PCR method using primers containing the primer (A) and the following primer (D) or (E) together with an amplification product obtained in the first step as a template,
(D) a primer, comprising a nucleotide sequence which is depicted as a portion or a whole of a sequence complementary to a region between nucleotides 124 to 156 of SEQ ID No.3 and has at least ten nucleotides in length;
(E) a primer, consisting of a nucleotide sequence which is depicted as a sequence complementary to a region between nucleotides 124 to 143 of SEQ ID No.3.

8. A method for detecting and/or quantifying Fusobacterium nucleatum, **characterized in that** the method comprises:
a first step of conducting a PCR method using primers containing the following primers (F)and (B),
(F) a primer, comprising a nucleotide sequence which is depicted as a portion of SEQ ID No. 4 and has at least ten nucleotides in length,
(B) a primer, comprising a nucleotide sequence which is depicted as a portion or a whole of a sequence complementary to a region between nucleotides 863 to 883 of SEQ ID No.3 and has at least ten nucleotides in length; and,
a second step of conducting a PCR method using primers containing the following primers (A) and (D) together with an amplification product obtained in the first step as a template.
(A) a primer, comprising a nucleotide sequence which is depicted as a portion or a whole of a region between nucleotides 1 to 39 of SEQ ID No.3 and has at least ten nucleotides in length,
(D) a primer, comprising a nucleotide sequence which is depicted as a portion or a whole of a sequence complementary to a region between nucleotides 124 to 156 of SEQ ID No.3 and has at least ten nucleotides in length.

9. A gene amplification product specific for Fusobacterium nucleatum obtained by conducting a PCR method, **characterized in that** the method uses the following primer
(A) and the following primer (D) or (E) together with a DNA containing a nucleotide sequence of SEQ ID No. 5 as a template.
(A) a primer, comprising a nucleotide sequence which is depicted as a portion or a whole of a region between nucleotides 1 to 39 of SEQ ID No.3 and has at least ten nucleotides in length;
(D) a primer, comprising a nucleotide sequence which is depicted as a portion or a whole of a sequence complementary to a region between nucleotides 124 to 156 of SEQ ID No.3 and has at least ten nucleotides in length;
(E) a primer, consisting of a nucleotide sequence which is depicted as a sequence complementary to a region between nucleotides 124 to 143 of SEQ ID No.3.

10. A probe having a nucleotide sequence depicted as SEQ ID No. 5.


SEQUENCE LISTING

<110> Kao Corporation

<120> Primers For Detecting Fusobacterium Nucleatum By Pcr Methods And Methods For Detection

<130> 020962EP

<150> JP 2002-358698
<151> 2002-12-10

<150> JP 2003-403715
<151> 2003-12-02

<160> 17

<170> PatentIn version 3.1

<210> 1
<211> 1661
<212> DNA
<213> Fusobacterium nucleatum

<220>
<221> misc_feature
<222> (1650)..(1650)
<223> n stands for any base

<400> 1
attgaacgaa gagtttgatc ctggctcagg atgaacgctg acagaatgct taacacatgc 60

aagtctactt gaatttgggt tttttaactt cgatttgggt ggcggacggg tgagtaacgc 120

gtaaagaact tgcctcacag ctagggacaa catttggaaa cgaatgctaa tacctgatat 180

tatgattata gggcatccta gaattatgaa agctatatgc gctgtgagag agctttgcgt 240

cccattagct agttggagag gtaacggctc accaaggcga tgatgggtag ccggcctgag 300

agggtgaacg gccacaaggg gactgagaca cggcccttac tcctacggga ggcagcagtg 360

gggaatattg gacaatggac cgagagtctg atccagcaat tctgtgtgca cgatgacgtt 420

tttcggaatg taaagtgctt tcagttggga agaaaaaaat gacggtacca acagaagaag 480

tgacggctaa atacgtgcca gcagccgcgg taatacgtat gtcacgagcg ttatccggat 540

ttattgggcg taaagcgcgt ctaggtggtt atgtaagtct gatgtgaaaa tgcagggctc 600

aactctgtat tgcgttggaa actgtgtaac tagagtactg gagaggtaag cggaactaca 660

agtgtagagg tgaaattcgt agatatttgt aggaatgccg atggggaagc cagcttactg 720

gacagatact gacgctgaag cgcgaaagcg tgggtagcaa acaggattag ataccctggt 780

agtccacgcc gtaaacgatg attactaggt gttgggggtc gaacctcagc gcccaagcaa 840

acgcgataag taatccgcct ggggagtacg tacgcaagta tgaaactcaa aggaattgac 900

ggggacccgc acaagcggtg gagcatgtgg tttaattcga cgcaacgcga ggaaccttac 960

cagcgtttga catcttagga atgagacaga gatgtttcag tgtcccttcg gggaaaccta 1020

aagacaggtg gtgcatggct gtcgtcagct cgtgtcgtga gatgttgggt taagtcccgc 1080

aacgagcgca acccctttcg tatgttacca tcattaagtt ggggactcat gcgatactgc 1140

ctacgatgag taggaggaag gtggggatga cgtcaagtca tcatgcccct tatacgctgg 1200

gctacacacg tgctacaatg ggtagaacag agagttgcaa agccgtgagg tggagctaat 1260

ctcagaaaac tattcttagt tcggattgta ctctgcaact cgagtacatg aagttggaat 1320

cgctagtaat cgcgaatcag caatgtcgcg gtgaatacgt tctcgggtct tgtacacacc 1380

gcccgtcaca ccacgagagt tggttgcacc tgaagtagca ggcctaaccg taaggaggga 1440

tgttccgagg gtgtgattag cgattggggt gaagtcgtaa caaggtatcc gtacgggaac 1500

gtgcggatgg atcacctcct ttctaaggag aatgtgtctt tctctattct attggtaatg 1560

ttcttacatt acttctgaac attggaaact atatagtaga acaaacaaga aaaaaattaa 1620

ctctaaacaa tttctttaga gttagcttgn caaaaaaata g 1661

<210> 2
<211> 2927
<212> DNA
<213> Fusobacterium nucleatum

<400> 2
gttaaaataa ttaagggcac acaaaggatg cctaggtagt aagagccgat gaaggacgtg 60

gtaagctgcg ataagcctag ataagttgca atcgaacgta agagtctagg atttccgaat 120

ggagcaatct attaagatgg agtcttaata cgaaagaggg aaccgcgtga actgaaacat 180

ctaagtaacg cgaggaaaag aaagtaaaaa cgatacccaa agtagcggcg agcgaactgg 240

gtcaagccta aaccttaaat atgtcaagga tacagccgtt gtatttaagg ggtagaggga 300

caaagtagtg aagaactgta agatattcaa tatagtgtat tgatgaatta gaattgtctg 360

gaaagatgaa ccgcagaagg tgaaagtcct gtataagtaa atccttacac atataacttt 420

gctcccaagt aacatggaac acgaggaatt ctgtgtgaat cagtgaggac caaatctcat 480

aaggctaaat actcttacta accgatagcg catagtaccg tgagggaaag gtgaaaagaa 540

cccctggagg ggagtgaaat agaacctgaa attgtgtgct tacaagcggt cagagcccat 600

ttgggtgatg gcgtgccttt tggagaatga tcctgcgagt tacgttaaac ggcgaggtta 660

agtataacgg agccgaaggg aaaccaagtc ttaatagggc gaattagtcg tttggcgtag 720

acgcgaaacc tggtgatcta aacctgtcca ggatgaagct gtggtaagac acagtggagg 780

tcctaaccca ccgccgttga aaagttgggg gatgaggtag gtttaggggt gaaaagccaa 840

tcgaaccagg agatagctcg ttctctccga aatgcatcta ggtgcagcct tgagtgttca 900

attatggggg tagagcactg aatgatctag ggggcatatt gcttactgaa atcaatcaaa 960

ctccgaatac cataatttat agctcaggag tgagactatg ggagttaact tccattgtca 1020

aaagggaaac aacccagacc accagctaag gtccctaatt ataactaagt gggaaaggag 1080

gtggagattc acaaacaact aggaggttgg cttagaagca gccatacctt taaagagtgc 1140

gtaatagctc actagtcgag agtctctgcg ccgacaatgt aacggggcta agttataaac 1200

cgaagctgtg gaatcctttt ggattggtag gagagcgttc tgtaggccgt tgaagaagaa 1260

gggtaaccga ctttggaggt atcagaagtg agaatgcagg aataagtagc gagaaagggg 1320

gcgagaatcc tcctcgccgg aagaccaagg ttttcagggt aaagcttgtc ttccctgagt 1380

aagccgggac ctaagcccag gctataatgc gtaggcgaat ggaaaacaga ttaatatttc 1440

tgtgccagtc atgtattgtg atggagggac gcagaagggt atgcgcgcgg acgaacggaa 1500

gtgtccgtag aagtatgtag ggtgacttag taggtaaatc cattaagtta aatctgaggt 1560

atgatataca gtcgtaagat gaatgcgcaa atcccacgct gccaagaaaa gcttctaacg 1620

ttaatatatg actgcccgta ctgtaaaccg acacaggtgg tcaggatgag aaatctaagg 1680

cggacaggct aactctcgtt aaggaactct gcaaaataac ctcgtaactt cgggagaaga 1740

ggagcccttg tgtgtgagta tacacgcgat acaaagcgca cgagggtcgc agtgaagagg 1800

ctcaagcaac tgtttaacaa aaacacaggt ctatgctaag ctgtaaggcg atgtatatgg 1860

gctgacacct gcccagtgct ggaaggttaa gaggaggagt gagagctccg aattgaagcc 1920

ccagtgaacg gcggccgtaa ctataacggt cctaaggtag cgaaattcct tgtcgggtaa 1980

gttccgacct gcacgaatgg tgtaatgatt tgagcgctgt cttgacggga ggcctggtga 2040

aattgtatta ccggtgaaga taccggttac ctacagtagg acggaaagac cccatggagc 2100

tttactgtag cttggtattg ggttttggca ttgcatgtat aggatagttg ggagactatg 2160

atgatatggc gctagctgta tcggagtcat cggtggaata ccaaccattc aatgctgaaa 2220

ttctaatctg tggtttgtag ccacggagac agtgctaggt gggcagtttg actggggcgg 2280

tcgcctccga aagagtaacg gaggcgttca aaggttctct caggttggat ggaaatcaac 2340

catagagtgc aatggcataa gagagcttga ctgcaagact gacgggtcga gcagatgcga 2400

aagcaggaca tagtgatccg gcgattccga atggaagggt cgtcgctcaa cggataaaag 2460

ctaccctggg gataacaggc tgatcctacc cgagagtcca tatcgacggt agggtttggc 2520

acctcgatgt cggctcatcg catcctgggg ctggagaagg tcccaagggt tgggctgttc 2580

gcccattaaa gcggtacgtg agctgggttc agaacgtcgt gagacagttc ggtccctatc 2640

cactgtaggc gttagaatat tgagaagacc tgtccttagt acgagaggac cgggatggac 2700

aaacctctga tgtaccagtt gtcacgccag tggcacagct gggtagtcac gtttggaata 2760

gataaccgct gaaagcatct aagcgggaaa ctaacttcaa gataagtatt ctttaagata 2820

ccttcgagcc taggaggttg ataggttggg ggtgtaagta cagcaatgta tttagctgac 2880

caatactaat tatcgaagtt ttaatctaat atctactata tagtttc 2927

<210> 3
<211> 883
<212> DNA
<213> Fusobacterium nucleatum

<220>
<221> misc_feature
<222> (153)..(153)
<223> n stands for any base

<400> 3
aacgtgcgga tggatcacct cctttctaag gagaatgtgt ctttctctat tctattggta 60

atgttcttac attacttctg aacattggaa actatatagt agaacaaaca agaaaaaaat 120

taactctaaa caatttcttt agagttagct tgncaaaaaa taggttaaaa taattaaggg 180

cacacaaagg atgcctaggt agtaagagcc gatgaaggac gtggtaagct gcgataagcc 240

tagataagtt gcaatcgaac gtaagagtct aggatttccg aatggagcaa tctattaaga 300

tggagtctta atacgaaaga gggaaccgcg tgaactgaaa catctaagta acgcgaggaa 360

aagaaagtaa aaacgatacc caaagtagcg gcgagcgaac tgggtcaagc ctaaacctta 420

aatatgtcaa ggatacagcc gttgtattta aggggtagag ggacaaagta gtgaagaact 480

gtaagatatt caatatagtg tattgatgaa ttagaattgt ctggaaagat gaaccgcaga 540

aggtgaaagt cctgtataag taaatcctta cacatataac tttgctccca agtaacatgg 600

aacacgagga attctgtgtg aatcagtgag gaccaaatct cataaggcta aatactctta 660

ctaaccgata gcgcatagta ccgtgaggga aaggtgaaaa gaacccctgg aggggagtga 720

aatagaacct gaaattgtgt gcttacaagc ggtcagagcc catttgggtg atggcgtgcc 780

ttttggagaa tgatcctgcg agttacgtta aacggcgagg ttaagtataa cggagccgaa 840

gggaaaccaa gtcttaatag ggcgaattag tcgtttggcg tag 883

<210> 4
<211> 1502
<212> DNA
<213> Fusobacterium nucleatum

<400> 4
attgaacgaa gagtttgatc ctggctcagg atgaacgctg acagaatgct taacacatgc 60

aagtctactt gaatttgggt tttttaactt cgatttgggt ggcggacggg tgagtaacgc 120

gtaaagaact tgcctcacag ctagggacaa catttggaaa cgaatgctaa tacctgatat 180

tatgattata gggcatccta gaattatgaa agctatatgc gctgtgagag agctttgcgt 240

cccattagct agttggagag gtaacggctc accaaggcga tgatgggtag ccggcctgag 300

agggtgaacg gccacaaggg gactgagaca cggcccttac tcctacggga ggcagcagtg 360

gggaatattg gacaatggac cgagagtctg atccagcaat tctgtgtgca cgatgacgtt 420

tttcggaatg taaagtgctt tcagttggga agaaaaaaat gacggtacca acagaagaag 480

tgacggctaa atacgtgcca gcagccgcgg taatacgtat gtcacgagcg ttatccggat 540

ttattgggcg taaagcgcgt ctaggtggtt atgtaagtct gatgtgaaaa tgcagggctc 600

aactctgtat tgcgttggaa actgtgtaac tagagtactg gagaggtaag cggaactaca 660

agtgtagagg tgaaattcgt agatatttgt aggaatgccg atggggaagc cagcttactg 720

gacagatact gacgctgaag cgcgaaagcg tgggtagcaa acaggattag ataccctggt 780

agtccacgcc gtaaacgatg attactaggt gttgggggtc gaacctcagc gcccaagcaa 840

acgcgataag taatccgcct ggggagtacg tacgcaagta tgaaactcaa aggaattgac 900

ggggacccgc acaagcggtg gagcatgtgg tttaattcga cgcaacgcga ggaaccttac 960

cagcgtttga catcttagga atgagacaga gatgtttcag tgtcccttcg ggaaacctaa 1020

aagacaggtg gtgcatggct gtcgtcagct cgtgtcgtga gatgttgggt taagtcccgc 1080

aacgagcgca acccctttcg tatgttacca tcattaagtt ggggactcat gcgatactgc 1140

ctacgatgag taggaggaag gtggggatga cgtcaagtca tcatgcccct tatacgctgg 1200

gctacacacg tgctacaatg ggtagaacag agagttgcaa agccgtgagg tggagctaat 1260

ctcagaaaac tattcttagt tcggattgta ctctgcaact cgagtacatg aagttggaat 1320

cgctagtaat cgcgaatcag caatgtcgcg gtgaatacgt tctcgggtct tgtacacacc 1380

gcccgtcaca ccacgagagt tggttgcacc tgaagtagca ggcctaaccg taaggaggga 1440

tgttccgagg gtgtgattag cgattggggt gaagtcgtaa caaggtatcc gtacgggaac 1500

gt 1502

<210> 5
<211> 152
<212> DNA
<213> Fusobacterium nucleatum

<400> 5
aacgtgcgga tggatcacct cctttctaag gagaatgtgt ctttctctat tctattggta 60

atgttcttac attacttctg aacattggaa actatatagt agaacaaaca agaaaaaaat 120

taactctaaa caatttcttt agagttagct tg                                    152

<210>  6
<211>  18
<212>  DNA
<213>  Fusobacterium nucleatum

<400>  6
aacgtgcgga tggatcac                                                    18

<210>  7
<211>  21
<212>  DNA
<213>  Fusobacterium nucleatum

<400>  7
ctacgccaaa cgactaattc g                                                21

<210>  8
<211>  21
<212>  DNA
<213>  Fusobacterium nucleatum

<400>  8
ggattagata ccctggtagt c                                                21

<210>  9
<211>  16
<212>  DNA
<213>  Fusobacterium nucleatum

<400>  9
gccatcaccc aaatgg                                                      16

<210>  10
<211>  20
<212>  DNA
<213>  Fusobacterium nucleatum

<400>  10
tctaaagaaa ttgtttagag                                                  20

<210>  11
<211>  17
<212>  DNA
<213>  Fusobacterium nucleatum

<400>  11
gtttgatcct ggctcag                                                     17

<210>  12
<211>  17
<212>  DNA
<213>  Fusobacterium nucleatum

<400>  12
cttaacacat gcaagtc                                                     17

<210>  13
<211>  21
<212>  DNA
<213>  Fusobacterium nucleatum

<400>  13
aatgcttaac acatgcaagt c                                                21

<210>  14

<211>  19
<212>  DNA
<213>  Fusobacterium nucleatum

<400>  14
tcctacggga ggcagcagt                                                    19


<210>  15
<211>  18
<212>  DNA
<213>  Fusobacterium nucleatum

<400>  15
gtcttgtaca caccgccc                                                     18


<210>  16
<211>  16
<212>  DNA
<213>  Fusobacterium nucleatum

<400>  16
gccatcaccc aaatgg                                                       16


<210>  17
<211>  18
<212>  DNA
<213>  Fusobacterium nucleatum

<400>  17
aagaagggta accgactt                                                     18